# EUROPÄISCHE PATENTANMELDUNG

(11) **EP 2 781 188 A1**
(43) Veröffentlichungstag der Anmeldung: **24.09.2014**
(21) Anmeldenummer: 14161056.8
(22) Anmeldetag: 21.03.2014
(51) Int. Cl.: A61B 5/01, A61F 7/12

(54) **Temperaturmessvorrichtung für intravaskuläre Anwendungen, Kühlkatheter mit einer Temperaturmessvorrichtung und Verfahren zur Temperaturmessung**

(30) Priorität: 22.03.2013 DE 102013102929
(71) Anmelder: Acandis GmbH & Co. KG, 76327 Pfinztal (DE)
(72) Erfinder: Cattaneo, Giorgio, 76199 Karlsruhe (DE)
(74) Vertreter: Kilchert, Jochen

(57) **Zusammenfassung**

Die Erfindung bezieht sich auf eine Temperaturmessvorrichtung für intravaskuläre Anwendungen auf einen Katheter 10 und wenigstens einen Temperaturmesssensor 11, der im Katheter 10 angeordnet ist. Sie zeichnet sich dadurch aus, dass der Katheter 10 untemperiert ist wobei der Temperaturmesssensor (11) einen Messfühler (12) und elektrische Leitungen (13) umfasst, die mit dem Messfühler (12) verbunden sind, wobei der Katheter (10) wenigstens ein Lumen (16, 17, 18), in dem mehrere elektrische Leitungen (13) angeordnet sind, und/oder wenigstens zwei Lumen (16, 17, 18) aufweist, in denen jeweils eine einzige elektrische Leitung (13) angeordnet ist, wobei der Katheter (10) einen Außendurchmesser von höchstens 0,9 mm aufweist.

## Beschreibung

Die Erfindung betrifft eine Temperaturmessvorrichtung für intravaskuläre Anwendungen, einen Kühlkatheter mit einer solchen Temperaturmessvorrichtung sowie ein Verfahren zur Temperaturmessung.

Bei der therapeutischen Hypothermie, insbesondere bei der Kühlung von Gehirngewebe bei Schlaganfallpatienten, ist die Messung der Temperatur in den betroffenen Arealen von entscheidender Bedeutung. Die gemessenen Temperaturwerte sind erforderlich, um die Parameter der Kühlung einzustellen. Bei einem Kühlkatheter wird beispielsweise dessen Leistung durch die Einstellung von Temperatur und/oder Strömungsmenge des Kühlmittels geregelt. Die Regelung kann automatisch oder manuell durch den Anwender erfolgen.

Ein Katheter mit einem Heizsystem zur Temperierung von Blut ist beispielsweise aus US 5,486,208 bekannt. Dieser Katheter weist eine Heizwendel im Bereich der Katheterspitze auf. Neben der Heizwendel ist ein Messsensor in das Kathetermaterial eingebettet, der die Temperatur der Katheterspitze misst, um die Versorgungsspannung der Heizwendel zu regeln. Ziel dieser Anordnung ist es, den Wärmeintrag in das Blut zu begrenzen, um eine Überhitzung zu vermeiden.

Eine Messung der Bluttemperatur ist mit den bekannten Kathetern nicht möglich. Vielmehr kann mit den bekannten Kathetern nur die Kathetertemperatur zur Bestimmung der Wärme gemessen werden, die vom Katheter auf das den Katheter umströmende Blut übertragen wird. Diese Messung ist ungenau, weil der Katheter in der Regel von der zu behandelnden Gefäßstelle proximal beabstandet ist. Im Umgebungsbereich des Katheters kommt es zu einer starken Wärmeübertragung, so dass sich die Bluttemperatur lokal entsprechend stark verändert. Mit zunehmendem Abstand vom Katheter erfolgt aber eine Durchmischung und damit eine Änderung der Temperatur des Blutes, die somit von der im Bereich des Katheters gemessenen Temperatur abweicht. Deshalb ist die Messung der Bluttemperatur im Bereich der Läsion mit dem bekannten Katheter ungenau. Gerade bei der Behandlung von Gehirnarealen oder generell bei der Behandlung kleiner Gefäße tritt dieses Problem auf, da der Katheter auf Grund seines großen Durchmessers relativ weit von der zu behandelnden Stelle beabstandet ist.

Dasselbe gilt für die Blutkühlung.

Der Erfindung liegt die Aufgabe zugrunde, eine Temperaturmessvorrichtung für intravaskuläre Anwendungen anzugeben, die für die therapeutische Hypothermie bei der Behandlung kleinlumiger Gefäße, wie beispielsweise im Gehirngewebe, geeignet ist. Der Erfindung liegt ferner die Aufgabe zugrunde, einen Kühlkatheter mit einer derartigen Temperaturmessvorrichtung sowie ein Verfahren zur Temperaturmessung anzugeben.

Erfindungsgemäß wird die Aufgabe mit Blick auf die Temperaturmessvorrichtung durch den Gegenstand des Anspruchs 1 gelöst. Hinsichtlich des Kühlkatheters und des Verfahrens zur Temperaturmessung wird die Aufgabe durch den Gegenstand des Anspruchs 14 bzw. des Anspruchs 16 gelöst.

Die Erfindung beruht auf dem Gedanken, eine Temperaturmessvorrichtung für intravaskuläre Anwendungen anzugeben, die einen Katheter und wenigstens einen Temperaturmesssensor aufweist, der im Katheter angeordnet ist. Der Katheter ist erfindungsgemäß untemperiert. Mit anderen Worten ist der Katheter ungeheizt und ungekühlt. Der Katheter enthält weder ein Heizsystem noch ein Kühlsystem, das eine Temperierung, d.h. eine Änderung der Temperatur des Blutes bewirkt. Der Katheter ist unbeheizbar und unkühlbar bzw. allgemein untemperierbar. Untemperiert bedeutet im Zusammenhang mit der Erfindung, dass der Katheter nicht beheizbar und nicht kühlbar ist in dem Sinne, dass dem Blut, das den Katheter umströmt, weder Wärme zuführbar ist noch dem Blut Wärme entzogen werden kann, um eine Änderung der Temperatur des Blutes herbeizuführen. Dies schließt nicht aus, dass dem Katheter Energie zuführbar ist, bspw. zum Betrieb des Temperaturmesssensors. Die zugeführte Energie, bspw. ein Messstrom, ist so gering, dass keine signifikante Erwärmung des Katheters erfolgt, der eine Temperaturänderung des Blutes bewirkt.

Dadurch wird erreicht, dass die Kühlung oder Erwärmung des Blutes einerseits und die Temperaturmessung andererseits getrennt, insbesondere räumlich getrennt, erfolgen. Die Wärmeübertragung und die Temperaturmessung sind durch die Erfindung entkoppelt.

Hinsichtlich der thermischen Eigenschaften des Katheters ist dessen Funktion auf die Temperaturmessung beschränkt. Die Erfindung führt daher zu einer Funktionstrennung, wobei die Blutkühlung bzw. Bluterwärmung einerseits und die Temperaturmessung andererseits durch verschiedene Katheter erfolgen. Die Katheter können abschnittsweise räumlich voneinander getrennt, d.h. konkret distal voneinander beabstandet, im zu behandelnden Blutgefäß bewegt werden.

Der Anwender hat damit die Möglichkeit, die Bluttemperatur an einer Stelle zu messen, die unabhängig von der Position des Katheters im Gefäß zur Kühlung bzw. Erwärmung des Blutes ist. Damit ist es möglich, das Blut proximal von der Läsion bzw. allgemein der zu behandelnden Stelle im Blutgefäß zu kühlen oder zu erwärmen und die Temperatur direkt an der zu behandelnden Stelle zu messen. Die Temperaturmessung mit Hilfe der erfindungsgemäßen Temperaturmessvorrichtung kann auch dazu genutzt werden, um die Wiedererwärmung des Blutes nach der Hypothermiebehandlung kontrolliert durchzuführen, um zu vermeiden, dass eine zu schnelle Erwärmung Nebeneffekte im Organismus hervorruft.

Dieser Vorteil kommt besonders bei kleinlumigen Gefäßen zum Tragen, wie beispielsweise bei cerebralen Gefäßen. Der Kühlkatheter bzw. generell der Temperierkatheter kann in einem proximalen Bereich des zu behandelnden Blutgefäßes positioniert werden, der einen großen Durchmesser hat. Dies hat den Vorteil, dass der Kühlkatheter größer als der Katheter der Temperaturmessvorrichtung dimensioniert ist und eine große Wärmeübertragungsfläche aufweist. Der untemperierte Katheter der erfindungsgemäßen Temperaturmessvorrichtung weist einen kleineren Außendurchmesser als der Temperierkatheter auf und kann in Gefäße vorgeschoben werden, die mit dem Temperierkatheter nicht erreichbar sind.

Insgesamt wird der Anwendungsbereich der therapeutischen Hypothermie auf Gefäße mit einem kleineren Durchmesser durch die Entkopplung der Temperaturmessung von der Wärmeübertragung erweitert.

Dabei wird sowohl die Temperaturmessvorrichtung für sich, also unabhängig von zusätzlichen Temperierkathetern offenbart und beansprucht. Außerdem wird eine Anordnung umfassend die erfindungsgemäße Temperaturmessvorrichtung und einen zusätzlichen Temperierkatheter offenbart und beansprucht.

Der Temperaturmesssensor weist einen Messfühler und elektrische Leitungen auf, die mit dem Messfühler verbunden sind. Der Katheter weist wenigstens ein Lumen, in dem mehrere elektrische Leitungen angeordnet sind, und/oder wenigstens zwei Lumen auf, in denen jeweils eine einzige elektrische Leitung angeordnet ist. Diese Ausführung hat fertigungstechnische Vorteile, weil die elektrischen Leitungen durch die/das Lumen des Katheters bei der Herstellung einfach hindurchgezogen werden können.

Bevorzugte Ausführungen der erfindungsgemäßen Temperaturmessvorrichtung sind in den Unteransprüchen angegeben.

Der Messfühler weist in der Regel zwei elektrische Leitungen auf. Die beiden Leitungen können einzeln in getrennten Lumen angeordnet sein (Einzellumen). Die Leitungen können in diesem Fall ohne elektrische Isolierung in den Lumen angeordnet sein, weil das Material des Katheters elektrisch isolierend wirkt. Alternativ können die Leitungen zusätzlich elektrisch isoliert sein. Die beiden Leitungen können auch in einem gemeinsamen Lumen angeordnet sein und sind dann elektrisch zu isolieren.

Wenn mehr als zwei elektrische Leitungen vorhanden sind, bspw. weil der Katheter mehr als einen Temperaturmesssensor aufweist, können zwei oder mehr gemeinsame Lumen, in denen jeweils zwei Leitungen geführt sind, im Katheter ausgebildet sein. Es ist auch möglich, wenigstens ein gemeinsames Lumen mit zwei oder mehr als zwei Einzellumen zu kombinieren.

Allgemein umfasst die elektrische Leitung einen Leiter aus einem elektrisch leitfähigen Leitermaterial bzw. die elektrische Leitung besteht aus einem Leiter aus einem elektrisch leitfähigen Leitermaterial.

Der Katheter kann zumindest abschnittsweise, insbesondere vollständig, aus einem Vollmaterial bestehen, in dem wenigstens zwei Lumen ausgebildet sind. Die Lumen bilden nebeneinander verlaufende, durch das Material des Katheters getrennte Kanäle, in denen die elektrischen Leitungen geführt sind. Die Lumen können als Einzellumen für jeweils eine einzige Leitung oder als gemeinsame Lumen für mehrere Leitungen fungieren.

Alternativ können die Leitungen in das Material des Katheters eingebettet sein.

Bei einer weiteren Ausführung ist der Katheter schlauchförmig ausgebildet und weist ein einziges Gesamtlumen auf, in dem alle elektrischen Leitungen angeordnet sind. Diese Ausführung bietet sich an, wenn der Temperaturmesssensor im Bereich der Katheterspitze angeordnet ist.

Vorzugsweise ist der Messfühler als Sensorkopf ausgebildet, der die distale Spitze des Katheters bildet, wobei der Sensorkopf einen Außendurchmesser von höchstens 0,5 mm, insbesondere höchstens 0,48 mm, insbesondere höchstens 0,47 mm aufweist und der Katheter einen Außendurchmesser von 0,6 mm bis 0,9 mm, insbesondere von 2,5 F, was einem Außendurchmesser von ca. 0,84 mm entspricht, aufweist. Diese Ausführung eignet sich besonders zur Anwendung mit dem als Schrumpfschlauch ausgeführten Katheter gemäß Anspruch 3 oder bei der Integrierung in 2,5 F Mikrokatheter.

Der Schrumpfschlauch kann den Sensorkopf umgeben, insbesondere vollständig umgeben.

Bei einer besonders bevorzugten Ausführung weist der Temperaturmesssensor, insbesondere der Messfühler, wenigstens eine Kontaktfläche für den Kontakt mit Blut auf, wobei die Kontaktfläche einen abgegrenzten Außenflächenabschnitt des Katheters bildet. Die abgegrenzte Kontaktfläche hat den Vorteil, dass der Wärmeübergang vom Blut auf den Messfühler unabhängig von anderen Eigenschaften des Katheters, wie Flexibilität oder Querschnittsstabilität, optimiert werden kann. Da die Kontaktfläche einen Außenflächenabschnitt des Katheters bildet, wird ein guter Wärmeübergang vom Blut auf den Temperaturmesssensor erreicht, da das Blut direkt in Kontakt mit der Kontaktfläche kommt.

Die Abgrenzung der Kontaktfläche kann bspw. dadurch erfolgen, dass der Katheter aus einem ersten Material und der Außenflächenabschnitt aus einem zweiten Material gebildet sind, das sich vom ersten Material unterscheidet. Die Abgrenzung erfolgt durch den Übergang von einem auf das andere Material. Mit anderen Worten besteht der Katheter aus mindestens zwei unterschiedlichen Materialien, wobei der Katheter hauptsächlich aus einem ersten Material gebildet ist, das die mechanischen Eigenschaften des Katheters bestimmt. Das zweite Material ist lokal auf die Kontaktfläche begrenzt und weist eine höhere Wärmeleitfähigkeit als das erste Material auf.

Die Kontaktfläche kann verschiedene geometrische Formen aufweisen.

Die Kontaktfläche kann seitlich in oder an einer Außenwand des Katheters ausgebildet sein. Dadurch wird ein abgegrenzter Bereich der Außenwand für die Temperaturmessung genutzt. Diese Ausführung ist platzsparend und effektiv, weil sich die Außenkontur des Katheters, an der das Blut vorbeiströmt, im Bereich der Kontaktfläche im Wesentlichen kontinuierlich fortsetzt. Die Außenkontur bleibt daher zumindest im Wesentlichen unverändert. Die Außenkontur kann bspw. zylindrisch sein.

Vorzugsweise bildet die Kontaktfläche eine distale Spitze des Katheters. Dadurch wird die gesamte Katheterlänge genutzt, um den Temperaturmesspunkt möglichst weit in distaler Richtung im Gefäß zu positionieren. Dabei kann auch die Bluttemperatur in der Gefäßmitte gemessen werden.

Um die Führung der Temperaturmessvorrichtung im Blutgefäß zu verbessern, kann ein Führungsdraht in Längsrichtung durch den Katheter bewegbar sein. Die Temperaturmessvorrichtung ist für den Gebrauch zusammen mit einem Führungsdraht angepasst. Konkret wird offenbart und beansprucht, dass der Führungsdraht in Längsrichtung bewegbar im Katheter angeordnet ist. Dabei kann beispielsweise der Führungsdraht in einem dritten Lumen, d.h. zusätzlich zu den beiden getrennten Lumen für die elektrischen Leitungen, oder im gemeinsamen Lumen angeordnet sein.

Vorzugsweise ist der im gemeinsamen Lumen angeordnete Führungsdraht fixiert. Insbesondere ist der im gemeinsamen Lumen angeordnete Führungsdraht durch den als Schrumpfschlauch ausgebildeten Katheter fixiert. Der Führungsdraht versteift den Katheter und verbessert so die Manövrierbarkeit, d.h. die Führung im Gefäß.

Die Handhabbarkeit des Katheters wird bei einer bevorzugten Ausführungsform dadurch verbessert, dass der Katheter im Bereich des distalen Endes flexibler als im Bereich des proximalen Endes ausgebildet ist. Dies hat den Vorteil, dass die axiale Stabilität des Katheters am proximalen Ende erhöht wird, um die Sicherheit beim Vorschieben des Katheters im Blutgefäß zu verbessern. Durch die im Vergleich zum proximalen Ende erhöhte Flexibilität des Katheters am distalen Ende wird erreicht, dass der Katheter und damit die Temperaturmessvorrichtung in gewundene Gefäße mit kleinen Krümmungsradien vorgeschoben werden kann.

Vorzugsweise weist der Katheter eine Umhüllung, insbesondere eine Kunststoffumhüllung auf. Durch die Umhüllung können die mechanischen Eigenschaften des Katheters gezielt verbessert werden. Es versteht sich, dass der Außenflächenabschnitt im Fall der Umhüllung deren Außenbegrenzung bildet.

Die Umhüllung kann in Längsrichtung unterschiedliche Härtegrade, insbesondere Shorehärtegrade, aufweisen. Am proximalen Ende kann der Katheter eine größere Härte als am distalen Ende aufweisen. Damit wird die Querschnittsstabilität verbessert und einer Deformation des Katheters bspw. beim Schieben in einem Führungskatheter entgegengewirkt. Im Bereich der Katheterspitze wird eine im Vergleich zum proximalen Ende größere Flexibilität erreicht, die das Navigieren im Gefäß erleichtert. Der Katheter kann mehr als zwei unterschiedliche Bereiche mit unterschiedlicher Härte aufweisen. Die Bereiche können kontinuierliche Übergänge der Härtegrade oder diskrete Übergänge aufweisen.

Der Katheter kann eine Metallverstärkung, insbesondere unterhalb der Umhüllung aufweisen. Die Metallverstärkung dient dazu, die axiale Stabilität zu verbessern. Im Bereich der Kontaktfläche kann die Metallverstärkung unterbrochen sein, d.h. der Bereich der Kontaktfläche kann verstärkungsfrei sein.

Der Temperatursensor, insbesondere der Messfühler kann Anschlussleitungen aufweisen, die mit dem Messfühler, insbesondere mit dem Sensorkopf verbunden sind. Im Sensorkopf ist ein mit den Anschlussleitungen verbundenes, wärmeempfindliches Sensorelement angeordnet.

Der Temperaturmesssensor, insbesondere der Messfühler kann wenigstens einen Messdraht aufweisen. Der Messdraht kann im Bereich der Kontaktfläche, insbesondere unterhalb und auf derselben Höhe wie die Kontaktfläche eine distale Messspitze bilden. Die Messspitze weist zwei proximale Drahtenden auf, die die elektrischen Leitungen des Temperaturmesssensors bilden. Die Messspitze ist im Messfühler, insbesondere im Sensorkopf angeordnet.

Bei einem Widerstandssensor bestehen der Messdraht und die Anschlussleitungen aus ein und demselben Material. Die Messpitze kann durch Umlenken eines durchgängigen Drahtes oder durch Verbinden zweier Einzeldrähte gebildet sein. Bei einem Thermoelement besteht der Messdraht aus zwei unterschiedlichen Drähten aus verschiedenen Materialien, die im Bereich der Messspitze verbunden sind. Die Messspitze ist mit den Anschlussleitungen verbunden.

Andere Sensorelemente sind möglich, bspw. NTC- oder PTC Sensorelemente (Heißleiter bzw. Kaltleiter). Bei einem drahtgewickelten PTC-Sensorelement ist ein Messdraht vorgesehen. Bei einem NTC-Sensorelement bildet ein Halbleiterbauteil das Sensorelement. PTC-Dünnschichtsensoren weisen ein platinbeschichtete Oberfläche auf.

Die Messspitze oder allgemein das Sensorelement ist in das Material der Kontaktfläche eingebettet, d.h. vollflächig vom Material der Kontaktfläche umgeben. Das Material der Kontaktfläche erstreckt sich radial nach innen und bildet zusammen mit der Messspitze oder allgemein mit dem Sensorelement einen Messblock im Katheter, der vom übrigen Kathetermaterial abgegrenzt ist. Diese Ausführung ist besonders für die seitliche Kontaktfläche geeignet.

Diese Ausführung hat den Vorteil, dass das den Messfühler umgebende Material mit Blick auf die Wärmeleiteigenschaften optimiert ist, um eine gute Wärmeübertragung zwischen dem Blut und dem Messdraht zu erreichen. Vorzugsweise weist der Katheter wenigstens zwei Temperaturmesssensoren auf. Diese können beispielsweise an unterschiedlichen Positionen am Katheter angeordnet sein. Damit ist es möglich, die Bluttemperatur an wenigstens zwei verschiedenen Messstellen zu erfassen. Dabei kann ein einzelner Temperaturmesssensor seitlich am Katheter und ein weiterer Temperaturmesssensor im Bereich der Katheterspitze angeordnet sein. Generell können die wenigstens zwei Temperaturmesssensoren axial in Längsrichtung des Katheters voneinander beabstandet angeordnet sein. Es ist auch möglich, mehrere Temperaturmesssensoren seitlich in Längsrichtung des Katheters und/oder seitlich auf dem Umfang des Katheters verteilt anzuordnen. Diese seitlich angeordneten Temperaturmesssensoren können mit einem Temperaturmesssensor im Bereich der Katheterspitze kombiniert werden.

Generell kann jeder Temperaturmesssensor eine eigene abgegrenzte Kontaktfläche aufweisen.

Es hat sich als zweckmäßig erwiesen, wenn die Katheterlänge wenigstens 0,6 m beträgt. Zu den weiteren Untergrenzen der Katheterlänge wird auf Anspruch 18 verwiesen. Der Außendurchmesser des Katheters kann höchstens 1,5 mm betragen. Zu den weiteren Obergrenzen für den Außendurchmesser wird auf Anspruch 19 verwiesen.

Nach dem nebengeordneten Anspruch 21 betrifft die Erfindung einen Kühlkatheter mit einer Temperaturmessvorrichtung, wie vorstehend beschrieben, und einem Kühlelement, wobei die Temperaturmessvorrichtung in einem Lumen des Kühlkatheters derart längsbeweglich angeordnet ist, dass der Temperaturmesssensor in distaler Richtung vom Kühlelement beabstandet angeordnet werden kann. Im Unterschied zum Stand der Technik ist bei diesem Kühlkatheter der Temperaturmesssensor nicht direkt in den Kühlkatheter, d.h. in das Material des Kühlkatheters integriert. Vielmehr ermöglicht die Erfindung durch die Entkopplung der Wärmeübertragung von der Temperaturmessung die Einstellung eines Abstandes zwischen dem Temperaturmesssensor und dem Kühlelement. Der Temperaturmesssensor bzw. allgemein die Temperaturmessvorrichtung ist dazu in Längsrichtung des Kühlkatheters relativ zum Kühlelement bewegbar. Dies hat den Vorteil, dass eine Durchschnittsbluttemperatur gemessen werden kann, die sich im Abstand zum Kühlelement nach einer gewissen Blutdurchmischung einstellt.

Um das Kühlelement herum bzw. in unmittelbarer Nähe des Kühlelements bildet sich eine Grenzschicht aus kalter Flüssigkeit direkt an der Wandung des Kühlkatheters, die die Bluttemperaturmessung in unmittelbarer Nähe zum Kühlkatheter verfälscht. Die längsbewegliche Anordnung der Temperaturmessvorrichtung in einem Lumen des Kühlkatheters ermöglicht die Positionierung des Temperaturmesssensors in einem solchen Abstand vom Kühlelement, dass die Durchschnittsbluttemperatur insbesondere im Bereich der zu behandelnden Stelle gemessen werden kann.

Vorzugsweise weist der Kühlkatheter ein expandierbares Rekanalisationselement im Katheter auf, das im Katheter derart längsbeweglich angeordnet ist, dass das Rekanalisationselement in distaler Richtung vom Kühlelement beabstandet angeordnet werden kann. Dabei können das Rekanalisationselement und die Temperaturmessvorrichtung relativ beweglich zueinander sein. Diese Ausführungsform des Kühlkatheters hat den Vorteil, dass die Funktionsteilung einerseits eine flexible Handhabung des Kühlkatheters und andererseits eine freie Positionierung der Temperaturmessstelle ermöglicht. Das expandierbare Rekanalisationselement kann vom Anwender distal zum Kühlelement beabstandet angeordnet werden, so dass das Kühlelement in einem Bereich des Blutgefäßes mit einem relativ großen Durchmesser für die Kühlung bzw. allgemeine Temperierung des Blutes sorgt. Die Temperaturmessvorrichtung kann im Bereich des Rekanalisationselements angeordnet werden, um direkt an der Behandlungsstelle die Bluttemperatur zu messen. Es ist auch möglich, die Temperaturmessvorrichtung proximal oder distal zum expandierten Rekanalisationselement zu positionieren, um dort die Temperatur zu messen.

Bei dem erfindungsgemäßen Verfahren zur intravaskulären Temperaturmessung von Blut wird eine Temperaturmessvorrichtung, wie oben beschrieben, durch einen Kühlkatheter so weit vorgeschoben, dass der Temperaturmesssensor distal von einem Kühlelement des Kühlkatheters positioniert wird.

Die Erfindung wird nachfolgend anhand von Ausführungsbeispielen unter Bezugnahme auf die beigefügten schematischen Figuren mit weiteren Einzelheiten erläutert.

In diesen zeigen:
- Figur 1: einen Querschnitt durch einen Katheter einer Temperaturmessvorrichtung nach einem erfindungsgemäßen Ausführungsbeispiel mit zwei Lumen für die Aufnahme von elektrischen Leitungen;
- Figur 2: einen Querschnitt durch einen Katheter, ähnlich wie in Figur 1, der zusätzlich ein drittes Lumen zur Aufnahme eines Führungsdrahtes aufweist;
- Figur 3: einen Querschnitt durch den Katheter gemäß Figur 2, der eine Metallverstärkung und eine Außenumhüllung aufweist;
- Figur 4: einen Querschnitt durch den Katheter gemäß Figur 2, der in einem Mikrokatheter angeordnet ist;
- Figur 5: einen Querschnitt durch einen Katheter einer Temperaturmessvorrichtung nach einem weiteren Ausführungsbeispiel, der ein gemeinsames Lumen zur Aufnahme der elektrischen Leitungen und des Führungsdrahtes aufweist;
- Figur 6: einen Längsschnitt durch den Katheter gemäß Figur 1;
- Figur 7: einen Längsschnitt durch einen Katheter einer Temperaturmessvorrichtung nach einem weiteren Ausführungsbeispiel, bei dem zwei Temperaturmesssensoren vorgesehen sind;
- Figur 8: einen Querschnitt durch einen Katheter einer Temperaturmessvorrichtung nach einem weiteren erfindungsgemäßen Ausführungsbeispiel, bei dem ein Führungsdraht im Katheter angeordnet ist;
- Figur 9: einen Längsschnitt durch einen Kühlkatheter mit einer Temperaturmessvorrichtung, die relativ zum Kühlkatheter bewegbar ist;
- Figur 10: eine schematische Anordnung eines Kühlkatheters mit einer Temperaturmessvorrichtung nach einem erfindungsgemäßen Ausführungsbeispiel im Blutgefäß; und
- Figur 11: eine schematische Ansicht eines Kühlkatheters mit Rekanalisationselement und einer Temperaturmessvorrichtung nach einem erfindungsgemäßen Ausführungsbeispiel in einem Blutgefäß.

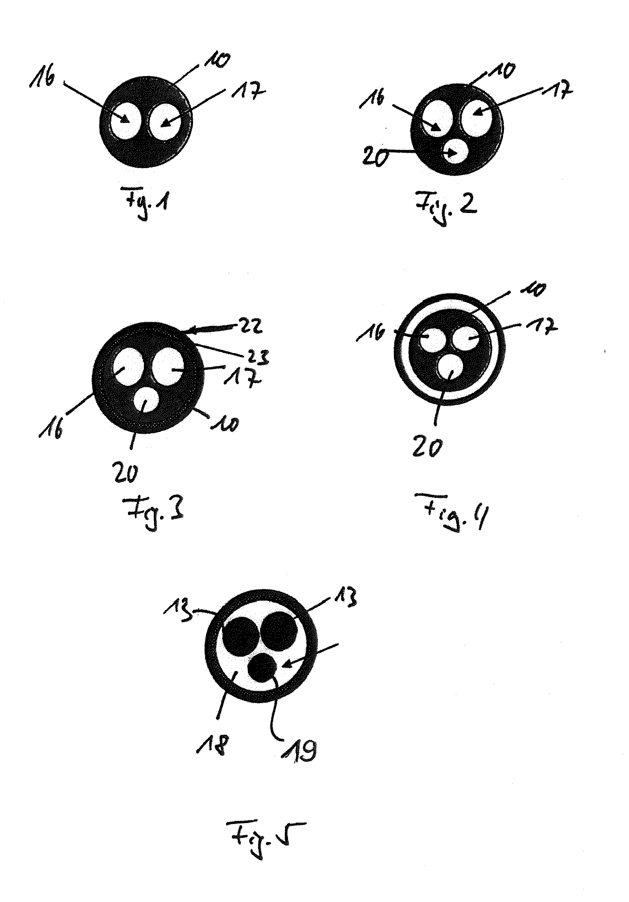

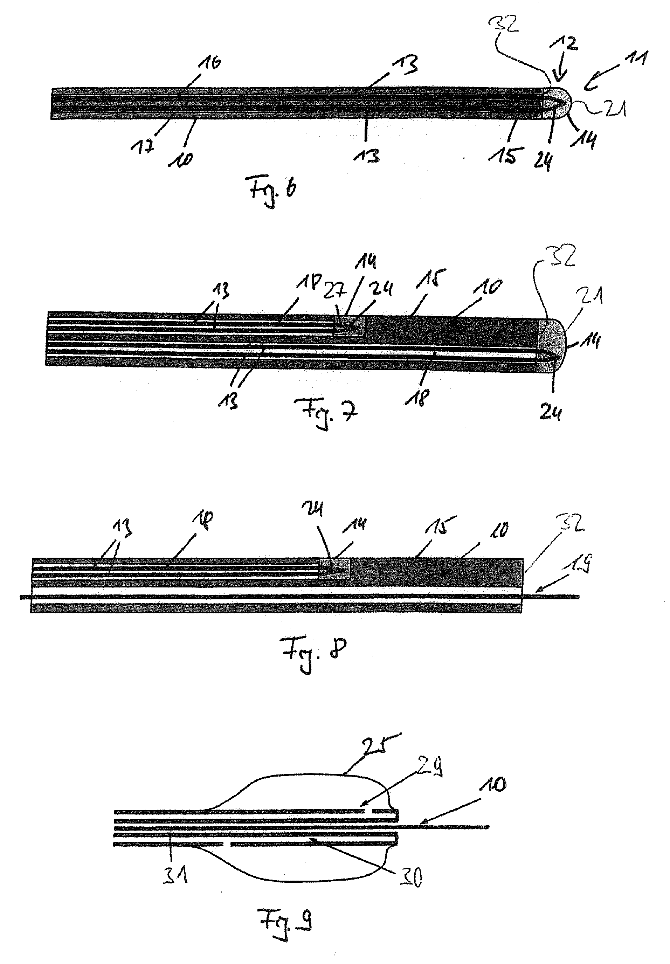

Ein Beispiel für eine Temperaturmessvorrichtung nach der Erfindung ist in den Figuren 1 und 6 dargestellt, wobei Figur 1 einen Querschnitt durch den Katheter 10 der Temperaturmessvorrichtung ohne elektrische Leitungen und Figur 6 den Katheter gemäß Figur 1 im Längsschnitt mit elektrischen Leitungen 13 darstellt. Figur 6 zeigt einen Abschnitt des Katheters im Bereich der distalen Spitze. Die elektrischen Leitungen 13 erstrecken sich entlang der gesamten Katheterlänge (nicht dargestellt) und enden am proximalen Ende mit Anschlüssen für eine Messeinrichtung (nicht dargestellt) bzw. für eine Stromversorgung (nicht dargestellt) bspw. bei einem Widerstandssensor.

Der gesamte Katheter der Temperaturmessvorrichtung gemäß Figur 6 ist untemperiert. Der Katheter ist vollständig frei von Heiz- und Kühleinrichtungen, die eine Wärmeübertragung zwischen dem Katheter 10 und dem Blut, das den Katheter 10 im Gebrauch umströmt, bewirken. Mit anderen Worten ist der Katheter weder beheizbar noch kühlbar. Die einzige Funktion des Katheters 10 besteht darin, eine Temperaturmessung zu ermöglichen. Dazu weist der Katheter 10 einen Temperaturmesssensor 11 auf, der im Katheter 10 angeordnet ist. Mehrere Temperaturmesssensoren sind möglich. Bis auf die Temperaturmesssensoren ist der Katheter 10 einbautenfrei.

Die vorstehenden Erläuterungen zur fehlenden Beheizbarkeit und zur fehlenden Kühlbarkeit des Katheters 10 werden im Zusammenhang mit allen Ausführungsbeispielen dieser Anmeldung offenbart und beansprucht.

Wie Figur 6 weiter zu entnehmen ist, weist der Temperaturmesssensor 11 einen Messfühler 12 auf. Der Messfühler 12 ist mit elektrischen Leitungen 13 verbunden, die im Betrieb des Katheters an eine Messeinrichtung mit oder ohne Stromversorgung angeschlossen sind. Mit anderen Worten sind die elektrischen Leitungen 13 mit einer Messeinrichtung verbindbar.

Die elektrischen Leitungen 13 sind im Katheter 10 geführt. Das kann beispielsweise, wie in Figur 6 dargestellt, dadurch erfolgen, dass im Katheter 10 zwei Lumen 16, 17 (siehe Figur 1) ausgebildet sind, die Leitungskanäle für die elektrischen Leitungen 13 bilden. Jeweils eine elektrische Leitung 13 ist in einem Lumen 16, 17 angeordnet. Wenn das Material des Katheters elektrisch isolierend ist, sind keine zusätzlichen Isolierungen der Leitungen 13 erforderlich. Alternativ können die Leitungen 13 jeweils für sich elektrisch isoliert sein.

Es ist auch möglich, die Leitungen 13 direkt in das Material des Katheters einzubetten. In diesem Fall stehen die Leitungen 13 jeweils vollumfänglich mit dem Kathetermaterial in Berührung.

Der Temperaturmesssensor 11, konkret der Messfühler 12 des Temperaturmesssensors 11, weist eine Kontaktfläche 14 auf. Diese Kontaktfläche 14 ist im Gebrauch des Katheters 10 in direktem Kontakt mit dem Blut, das am Katheter 10 vorbeiströmt, um einen guten Wärmeübergang zwischen dem Messfühler 12 und dem Blut zu erreichen. Dazu bildet die Kontaktfläche 14 einen Außenflächenabschnitt, der durch das Material vom Katheter abgegrenzt ist. Konkret bildet die Kontaktfläche 14 die Außenfläche eines Messblocks 27, in den der Messfühler 12 eingebettet ist. Der Messblock 27 und damit die Kontaktfläche 14 sind aus einem anderen Material als der Katheter 10 hergestellt, bspw. aus einem Harz, insbesondere einem Epoxidharz oder Keramik oder Kunststoff, bspw. PTFE. Dies gilt allgemein für alle Ausführungsbeispiele.

Bei dem Ausführungsbeispiel gemäß Figur 6 bildet die Kontaktfläche 14 bzw. der gesamte Messblock 27 die distale Katheterspitze 21, in der der Messfühler 12 angeordnet ist. Die Katheterspitze 21 ist halbkugelförmig und damit atraumatisch ausgebildet. Wenn für die Katheterspitze 21 ein hartes Material, bspw. Epoxidharz, verwendet wird, werden die Gleiteigenschaften verbessert. Bei einem weichen Material werden die Navigierbarkeit im Gefäß und die atraumatischen Eigenschaften verbessert.

Der Temperaturmesssensor 11 umfasst den Messfühler 12, die elekrischen Leitungen 13 sowie den Messblock 27 mit der Kontaktfläche 14.

Für alle Ausführungsbeispiele gilt, dass der Temperaturmesssensor 11 beispielsweise ein Thermoelement oder einen Widerstandssensor aufweist. Bei dem an sich bekannten Thermoelement sind zwei Leiter aus unterschiedlichen Metallen, bspw. die Materialpaarung Nickel / Nickel-Chrom, an deren Ende verbunden. Das Thermoelement wandelt Wärme in elektrische Energie um. Die dabei erzeugte elektrische Spannung wird für die Temperaturmessung genutzt. Derartige Thermoelemente sind dem Fachmann bekannt. Bei den ebenfalls an sich bekannten Widerstandssensoren wird die Temperaturabhängigkeit des elektrischen Widerstandes der verwendeten Leiter zur Messung der Temperatur genutzt. Einsetzbare PT100- oder Pt1000-Sensoren sind Messfühler, die als Messeffekt die Abhängigkeit des elektrischen Widerstands von der Temperatur bei Platin anwenden. Auch derartige Widerstandsmesssensoren sind dem Fachmann bekannt ebenso wir PTC- oder NTC-Sensoren.

Konkret weist der Messfühler 12 einen Messdraht 24 auf. Dieser kann einteilig oder zweiteilig ausgeführt sein. Bei einem Widerstandssensor, bei dem der Messdraht 24 aus demselben Material hergestellt ist, sind beide Varianten möglich. Bei einem Thermoelement bei dem zwei verschiedene Drahtmaterialien verwendet werden, ist die zweiteilige Variante möglich. Die zweiteilige Variante kann auch als Messfühler mit zwei Messdrähten 24 angesehen werden.

Der Messdraht 24 bildet eine distale Messspitze. Die proximalen Enden der Messspitze sind mit den elektrischen Leitungen 13, bspw. Kupferleiter, verbunden oder bilden jeweils einteilig die elektrischen Leitungen 13, wenn sich die proximalen Enden der Messspitze bis zum proximalen Ende des Katheters 10 erstrecken.

Der Messdraht 24 in den Messblock 27 eingebettet. Der Messblock 27 kann durch einen Klebstoff oder ein Harz, beispielsweise Epoxidharz, gebildet sein. Die unterschiedlichen Drähte des Thermoelements, bspw. aus Nickel und einer Nickel-Chromlegierung, oder der Platindraht bei PT100- bzw. PT1000-Sensoren können jeweils entsprechend eingebettet sein. Alternativ kann ein Kunststoff verwendet werden, der sich vom Kunststoff des Katheters unterscheidet.

Die Verwendung von Thermoelementen bzw. Widerstandsmessfühlern, wie vorstehend beschrieben, wird im Zusammenhang mit allen Ausführungsbeispielen offenbart und beansprucht.

Eine Variante des Katheters 10 gemäß Figur 1 bzw. gemäß Figur 6 ist in Figur 2 dargestellt, die zur Verbesserung der Führung der Temperaturmessvorrichtung im Blutgefäß ein drittes Lumen 20 für einen Führungsdraht aufweist. Das dritte Lumen 20 verläuft im Wesentlichen parallel zu den Lumen 16, 17 für die Aufnahme der elektrischen Leitungen 13. Ein weiteres Ausführungsbeispiel mit Führungsdraht 19 ist in Figur 8 dargestellt. Bei diesem Ausführungsbeispiel gemäß Figur 8 ist der Temperaturmesssensor 11 seitlich an der Außenwand bzw. in der Außenwand 15 des Katheters 10 angeordnet. Die Spitze des Katheters ist frei und weist eine Austrittsöffnung für den Führungsdraht 19 auf. Es ist auch möglich, den Katheter gemäß Figur 6 mit einem Führungsdraht bzw. einem dritten Lumen 20 für den Führungsdraht auszustatten. In diesem Fall tritt der Führungsdraht 19 seitlich neben der Kontaktfläche 14 des Temperaturmesssensors aus dem distalen Ende des Katheters 10 aus (nicht dargestellt).

Weitere Möglichkeiten, wie die elektrischen Leitungen 13 im Katheter 10 angeordnet sein können, zeigen die Figuren 5, 7 und 8. In den Figuren 7 und 8 ist dargestellt, dass zwei Leitungen 13 in ein und demselben Lumen 18, d.h. in einem gemeinsamen Lumen 18, angeordnet sind. In diesem Fall sind die Leitungen 13 elektrisch voneinander isoliert. Bei den Beispielen gemäß Figuren 6, 7 und 8 sowie bei den Beispielen gemäß Figuren 1 bis 4 ist der Katheter aus einem Vollmaterial gebildet, in dem wenigstens ein Lumen insbesondere mehrere Lumen 16, 17, 18, ausgebildet sind.

Im Unterschied dazu ist bei dem Katheter gemäß Figur 5 ein einziges gemeinsames Lumen 18 vorgesehen, in dem die elektrischen Leitungen 13 und der Führungsdraht 19 angeordnet sind. Bei dem Beispiel gemäß Figur 5 bildet der Katheter 10 eine schlauchförmige Durchführung, in der alle Leitungen 13 und der Führungsdraht 19 angeordnet sind.

Der Führungsdraht 19 kann dabei entweder relativ zum Katheter beweglich oder im Katheter fixiert sein. Im zweiten Fall kann der Katheter die drei oder mehr Elemente, d.h. die Leitungen 13 und den Draht 19 kraftschlüssig oder stoffschlüssig halten, insbesondere zusammenhalten. Für diesen Zweck wird der Katheter zB geschrumpft. Das gleiche gilt für den Fall, dass nur Leitungen 13 und kein drittes Element, wie zB der Führungsdraht, im Katheter angeordnet sind.

Der Katheter ist auf die Leitungen 13, insbesondere auf die Leitungen 13 und den Draht 14 aufgeschrumpft und liegt eng an diesen an. Der Katheter ist als Schrumpfschlauch ausgebildet.

Der fixierte Draht bildet einen Führungsdraht, weil dieser den Katheter versteift und so die Navigation in den Gefäßen erleichtert. Die Spitze des Drahtes kann bspw. gebogen sein.

Die Zwischenräume, die in Figur 5 zu sehen sind, verschwinden oder reduzieren sich im Volumen stark, wenn der Katheter bzw. Schrumpfschlauch aufgeschrumpft wird. Die Außenkontur kann von der runden Form abweichen. Bspw. kann sich eine ovale (bei zwei Leitungen) oder eine dreieckig/abgerundte From (bei drei Leitungen) einstellen.

Wie in den Figuren 6 bis 8 dargestellt, befindet sich die Kontaktfläche 14 des Temperaturmesssensors 11 in oder an der Außenwand 15 des Katheters 10. Die Kontaktfläche 14 kann seitlich in oder an der Außenwand 15 des Katheters 10 und/oder axial an der Außenwand 15 des Katheters 10 angeordnet sein. Die Kombination der seitlichen und axialen Anordnung ist in Fig. 7 gezeigt.

In einer weiteren Ausführung kann der Katheter sowohl die beiden isolierten Leitungen 13 als auch den Sensorkopf bzw. den Messfühler 12 umschließen. Der Katheter ummantelt die Leitungen 13 und den Sensorkopf bzw. den Messfühler 12. Der Katheter bzw. Schrumpfschlauch ist dazu aufgeschrumpft. Es ist möglich, dass der Katheter an der Spitze so weit geschrumpft wird, dass der Sensorkopf bzw. der Messfühler 12 distal abgedichtet ist. In diesem Fall ist das Material an der Kontaktfläche mit dem Blut entlang des gesamten Katheters und daher auch im Bereich des Sensorkopfs bzw. des Messfühlers 12 dasselbe.

Als Sensorkopf ist allgemein ein Messfühler zu verstehen, der ein Ende, d.h. einen Kopf einer Messvorrichtung bildet. Dies ist bspw. bei den Ausgestaltungen gemäß Fig. 6-8 der Fall, bei denen der Messfühler 12 bzw. Sensorkopf mit Anschlussleitungen verbunden ist. Im Messfühler 12 bzw. Sensorkopf ist ein Sensorelement integriert, bspw. ein NTC-Sensor, der in ein Kunststoffröhrchen mit Epoxidharz eingegossen ist. Andere Sensorelemente sind möglich.

Ein Beispiel für einen Katheter 10, bei dem die Kontaktfläche 14 axial an der Außenwand 15 angeordnet ist, ist in den Figuren 6 und 7 dargestellt. In diesem Fall bildet die Kontaktfläche 14 die distale Katheterspitze 21, sich in axialer Richtung ausgehend von der Stirnfläche 32 erstreckt. Die Katheterspitze 21 ist mit der Stirnfläche 32 verbunden.

In den Figuren 7 und 8 ist überdies gezeigt, dass die Kontaktfläche 14 seitlich in der Außenwand 15 ausgebildet ist. Die Kontaktfläche 14 bildet einen sich in Umfangs- und Längsrichtung des Katheters 10 erstreckenden Teil der Außenwand 15 und ist von dieser abgegrenzt. Die Abgrenzung der Kontaktfläche 14 von der Außenwand 15 kann beispielsweise dadurch erfolgen, dass die Kontaktfläche 14 aus einem anderen Material gebildet ist als die übrige Außenwand 15 des Katheters 10. Konkret kann der Bereich des Temperaturmesssensors 11, in dem der Messfühler 12 eingebettet ist, aus einem anderen Material bestehen, als das Material des Katheters 10. Dies ist in den Figuren 6 bis 8 durch die unterschiedlichen Grautöne des Katheters 10 und des Bereichs dargestellt, in dem der Messfühler 12 eingebettet ist.

Dieser Bereich bildet den Messblock 27, der sich radial nach innen in den Katheter 10 hinein erstreckt und vom Material des Katheters abgegrenzt ist, beispielsweise durch Wahl eines anderen Materials. Der Messblock 27 erstreckt sich so weit radial nach innen, dass der gesamte Messfühler 12 im Material des Messblocks 27 eingebettet ist. Die Außenfläche des Messblocks 27 bildet die Kontaktfläche 14. Die Herstellung des Messblockes kann durch Ausschneiden einer entsprechenden Ausnehmung aus der Außenwand 15 erfolgen, die nach der Montage des Messfühlers 14 mit einem Material, bspw. einem Harz, ausgegossen wird. Der Messblock kann die Form eines Zylinders aufweisen, der radial im Katheter 10 angeordnet ist. Andere Formen sind möglich.

Wie in Figur 7 dargestellt, kann die Temperaturmessvorrichtung zwei Temperatursensoren 11 aufweisen. Es ist auch möglich, mehr als zwei, beispielsweise drei oder vier oder mehr als vier Temperaturmesssensoren 12 vorzusehen. Konkret sind die Messfühler 14 der Temperaturmesssensoren 11 in axialer Richtung von einander beanstandet, so dass an unterschiedlichen Längspositionen des Katheters 10 Temperaturmessstellen ausgebildet sind. Bei dem Beispiel gemäß Figur 7 ist eine erste Temperaturmessstelle im Bereich der distalen Katheterspitze und eine zweite Temperaturmessstelle proximal beabstandet von der distalen Katheterspitze vorgesehen. Die proximal beabstandete Temperaturmessstelle wird durch einen seitlichen Messfühler 12 gebildet, dessen Kontaktfläche 14 mit der Außenwand 15 des Katheters fluchtet.

Es ist möglich, in Längsrichtung des Katheters 10 mehrere seitlich angeordnete Temperaturmesssensoren 11 vorzusehen, die jeweils einen Messfühler 12 und elektrische Leitungen 13 aufweisen. Die Messfühler 12 sind dabei sowohl axial als auch in Umfangsrichtung voneinander beabstandet.

Um die Manövrierbarkeit des Katheters 10 im Blutgefäß zu verbessern, kann dieser, wie in Figur 3 dargestellt, eine Umhüllung 22 aufweisen. Unterhalb der Umhüllung 22 ist bei dem Katheter gemäß Figur 3 eine Metallverstärkung 23 vorgesehen, d.h. radial innen, bezogen auf die Umhüllung 22. Dadurch wird die Stabilität des Katheters verbessert. Überdies ist es möglich, die Flexibilität des Katheters in Längsrichtung zu variieren. Dabei kann die Flexibilität im Bereich des proximalen Endes niedriger sein als die Flexibilität am distalen Ende, um einerseits die axiale Stabilität am proximalen Ende zu erhöhen und andererseits eine gute Anpassung des distalen Endes des Katheters an den Gefäßverlauf zu erreichen. Die vorstehend erläuterte Ausgestaltung der Außenhülle des Katheters wird im Zusammenhang mit allen Ausführungsbeispielen dieser Anmeldung offenbart. Besonders bevorzugt ist die Umhüllung 22 und die Metallverstärkung 23, wenn der Katheter 10 direkt in das Blutgefäß eingeführt wird, also ohne Führungskatheter.

Die Umhüllung 22 kann direkt mit dem Katheter 10 verbunden sein, d.h. ohne eine Zwischenschicht, wie die Metallverstärkung.

Die Umhüllung 22 kann auf den Katheter 10 aufgeschrumpft sein.

Es ist möglich, wie in Figur 4 gezeigt, den Katheter 10 der Temperaturmessvorrichtung mit Hilfe eines zweiten Außenkatheters 28 bzw. Führungskatheters zu verwenden, wobei der Katheter 10 im Außenkatheter 28 längsverschieblich angeordnet ist. Bei dem Außenkatheter 28 kann es sich um einen Mikrokatheter handeln. In diesem Fall ist keine Umhüllung erforderlich.

Die Länge des Katheters 10 beträgt wenigstens 0,6m, insbesondere wenigstens 0,7m, insbesondere wenigstens 0,8m, insbesondere wenigstens 0,9m, insbesondere wenigstens 1,0m, insbesondere wenigstens 1,1m, insbesondere wenigstens 1,2m. Für intracerebrale Anwendungen ist eine Länge von wenigstens 1,3m, insbesondere wenigstens 1,4m, insbesondere wenigstens 1,5m vorteilhaft. Die Obergrenze der Katheterlänge kann 1,6m betragen.

Der Außendurchmesser des Katheters 10 der Temperaturmessvorrichtung beträgt höchstens 1,5mm, insbesondere höchstens 1,2mm. Für intracerebrale Anwendungen beträgt der Außendurchmesser höchstens 1,0mm. Erfindungsgemäß beträgt der Außendurchmesser höchstens 0,9mm, insbesondere höchstens 0,8mm, insbesondere höchstens 0,7mm, insbesondere höchstens 0,6mm, insbesondere höchstens 0,5mm, insbesondere höchstens 0,4mm, insbesondere höchstens 0,3mm. Die Untergrenze kann 0,2mm betragen. Der Außendurchmesser entspricht dem Gesamtdurchmesser des Katheters, also einschließlich etwaiger Metallverstärkungen oder Umhüllungen. Die vorstehend genannten Dimensionen werden im Zusammenhang mit allen Ausführungsbeispielen der Erfindung offenbart. Der Gesamtdurchmesser, wie vorstehend offenbart, ermöglicht eine Führung der Temperaturmessvorrichtung mittels herkömmlicher Führungskatheter oder Mikrokatheter. Dabei kann es sich um einen 2F, 2,5F oder 3F Mikrokatheter handeln. Der Außendurchmesser eines 3F (french) Katheters beträgt ca. 1mm.

Wenn der Katheter 10 in Form eines Schlauches ohne zusätzliche Umhüllung und Metallverstärkung durch einen Mikrokatheter, wie in Figur 4, geschoben wird, weist der Schlauch einen maximalen Außendurchmesser von höchstens 1,2mm, insbesondere höchstens 1,1mm, insbesondere höchstens 1,0mm, insbesondere höchstens 0,8mm, insbesondere höchstens 0,7 mm, insbesondere höchstens 0,6mm, insbesondere höchstens 0,5mm, insbesondere höchstens 0,4mm, insbesondere höchstens 0,35mm, insbesondere höchstens 0,3mm, auf.

Der Messsensor hat allgemein einen Messfehler von höchstens 0,2°, höchstens 0,1°, höchstens 0,05°.

In Figur 9 ist ein Beispiel für einen Kühlkatheter dargestellt, der zusammen mit einer Temperaturmessvorrichtung nach einem der vorstehend genannten erfindungsgemäßen Ausführungsbeispiele verwendet wird. Der Kühlkatheter weist ein Kühlelement 25 auf, das im Bereich des distalen Endes des Kühlkatheters angeordnet ist. Bei dem Kühlelement 25 kann es sich beispielsweise um einen inflatierbaren Ballon handeln. Andere Kühlelemente sind möglich.

Der Kühlkatheter weist in an sich bekannter Weise ein Vorlauflumen 29 auf, das durch eine seitliche Öffnung mit dem Kühlelement 25 fluidverbunden ist. Der Kühlkatheter weist ferner ein Rücklauflumen 30 auf, das ebenfalls durch eine seitliche Öffnung mit dem Kühlelement 25 fluidverbunden ist. Wie in Figur 9 dargestellt, ist die Mündung des Vorlauflumens 29 distal zur Mündung des Rücklauflumens 30 angeordnet. Der Kühlkatheter weist ein Führungslumen 31 auf, in dem die Temperaturmessvorrichtung angeordnet ist. Konkret ist der Katheter 10 der Temperaturmessvorrichtung in dem Führungslumen 31 längsverschieblich angeordnet. Das Führungslumen kann zur Aufnahme eines Führungsdrahtes zum Positionieren des Kühlkatheters verwendet werden. Nach erfolgter Positionierung wird der Führungsdraht entfernt und der Katheter 10 der Temperaturmessvorrichtung in das Führungslumen 31 eingeführt. Wie in den Figuren 6 bis 8 dargestellt, ist der Messfühler 12 des Temperaturmesssensors im Bereich der distalen Spitze des Katheters 10 angeordnet. Durch eine Relativbewegung zwischen dem Katheter 10 und dem Kühlelement 25 kann der Messfühler 12 in distaler Richtung vom Kühlelement 25 beabstandet werden. Damit kann die Durchschnittsbluttemperatur gemessen werden, die sich nach einer Durchmischung des Blutes distal vom Kühlelement 25 einstellt. Die Länge des Kühlkatheters und des Katheters 10 der Temperaturmessvorrichtung kann jeweils so bemessen sein, dass im Gebrauch ein Abstand zwischen der Spitze des Kühlkatheters und der Spitze 21 des Katheters 10 der Temperaturmessvorrichtung wenigstens 5 cm, insbesondere wenigstens 10 cm, insbesondere wenigstens 15 cm, insbesondere wenigstens 20 cm, insbesondere wenigstens 25 cm, insbesondere wenigstens 30 cm beträgt. Der maximale Abstand kann 35 cm betragen. Die Längendifferenz zwischen dem Kühlkatheter und dem Katheter 10 der Temperaturmessvorrichtung kann wenigstens 10 mm, insbesondere wenigstens 15 mm, insbesondere wenigstens 20 mm, insbesondere wenigstens 25 mm, insbesondere wenigstens 30 mm, insbesondere wenigstens 40 mm betragen.

Der in Figur 9 dargestellte Kühlkatheter kann jeweils mit den in Figuren 6 bis 8 dargestellten Kathetern benutzt werden, die jeweils im Zusammenhang mit dem Kühlkatheter gemäß Figur 9 offenbart und beansprucht werden.

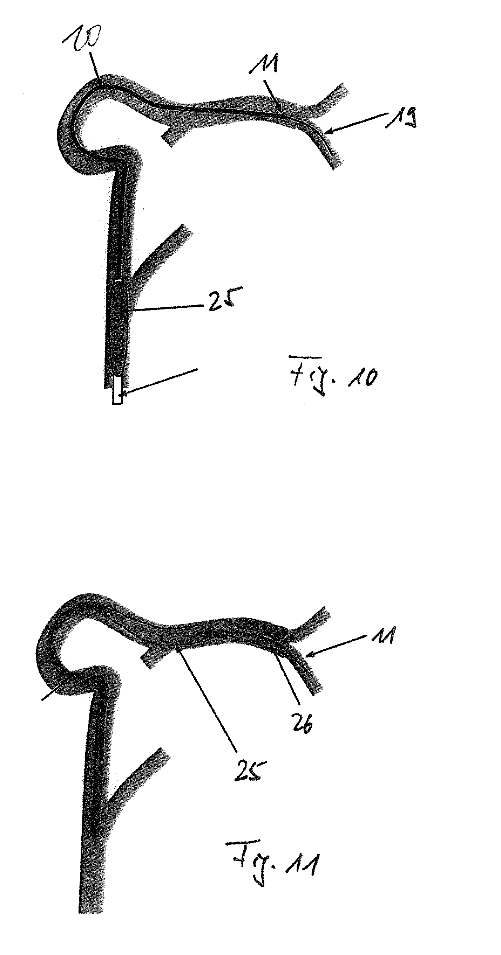

Ein Beispiel für die Anwendung des Kühlkatheters gemäß Figur 9 ist in Figur 10 dargestellt. Darin ist gezeigt, dass der Kühlkatheter mit dem Kühlelement 25 im Blutgefäß positioniert ist. Die Temperaturmessvorrichtung ist mittels des Führungsdrahtes 19 in distaler Richtung über das Kühlelement 25 hinaus vorgeschoben, wobei der Führungsdraht 19 bspw. hilft, die Temperaturmessvorrichtung an Bifurkationsstellen zu manövrieren. Wie in Figur 10 dargestellt, befindet sich der Temperaturmesssensor bzw. die Temperaturmesssensoren 11 im Bereich des distalen Endes des Katheters 10. Damit wird die Durchschnittsbluttemperatur in einem, bezogen auf das Kühlelement 25, distalen Bereich des Blutgefäßes gemessen.

Wie in Figur 11 dargestellt, kann die Temperaturmessvorrichtung zusammen mit einem Kühlkatheter und einer Rekanalisationseinrichtung 26 verwendet werden. Dabei sind sowohl das Rekanalisationselement 26 als auch der Katheter 10 der Temperaturmessvorrichtung relativ zueinander axialverschieblich. Dies kann beispielsweise dadurch erreicht werden, dass im Kühlkatheter zwei Lumen vorhanden sind, wobei im ersten Lumen das Rekanalisationselement 26 und im zweiten Lumen der Katheter 10 der Temperaturmessvorrichtung angeordnet sind. Dadurch ist eine Messung distal zum Thrombus während der Rekanalisation möglich. Die Temperaturmessvorrichtung kann mit oder ohne Führungsdraht 19 verwendet werden. Zum Einfädeln des Führungsdrahtes 19 kann der Katheter 10 der Temperaturmessvorrichtung einen Luer-Konnektor aufweisen.

### Bezugszeichenliste

- 10: Katheter
- 11: Temperaturmesssensor
- 12: Messfühler
- 13: Elektrische Leitungen
- 14: Kontaktfläche
- 15: Außenwand
- 16, 17, 18: Lumen
- 19: Führungsdraht
- 20: Drittes Lumen
- 21: Katheterspitze
- 22: Umhüllung
- 23: Metallverstärkung
- 24: Messdraht
- 25: Kühlelement
- 26: Rekanalisationselement
- 27: Messblock
- 28: Außenkatheter
- 29: Vorlauflumen
- 30: Rücklauflumen
- 31: Führungslumen
- 32: Stirnfläche

## Patentansprüche

1. Temperaturmessvorrichtung für intravaskuläre Anwendungen aufweisend einen Katheter (10) und wenigstens einen Temperaturmesssensor (11), der im Katheter (10) angeordnet ist,
**dadurch gekennzeichnet, dass**
der Katheter (10) untemperiert ist, wobei der Temperaturmesssensor (11) einen Messfühler (12) und elektrische Leitungen (13) umfasst, die mit dem Messfühler (12) verbunden sind, wobei der Katheter (10) wenigstens ein Lumen (16, 17, 18), in dem mehrere elektrische Leitungen (13) angeordnet sind, und/oder wenigstens zwei Lumen (16, 17, 18) aufweist, in denen jeweils eine einzige elektrische Leitung (13) angeordnet ist, wobei der Katheter (10) einen Außendurchmesser von höchstens 0,9 mm aufweist.

2. Temperaturmessvorrichtung nach Anspruch 1,
**dadurch gekennzeichnet, dass**
der Katheter (10) zumindest abschnittsweise, insbesondere vollständig aus einem Vollmaterial besteht, in dem wenigstens zwei Lumen (16, 17) ausgebildet sind.

3. Temperaturmessvorrichtung nach Anspruch 1,
**dadurch gekennzeichnet, dass**
der Katheter (10) schlauchförmig ausgebildet ist und ein einziges Gesamtlumen (18) aufweist, in dem alle elektrischen Leitungen (13) angeordnet sind, wobei der Katheter (10) insbesondere als Schrumpfschlauch ausgebildet ist.

4. Temperaturmessvorrichtung nach einem der vorhergehenden Ansprüche,
**dadurch gekennzeichnet, dass**
der Messfühler (12) als Sensorkopf ausgebildet ist und die distale Spitze (21) des Katheters (10) bildet, wobei der Sensorkopf einen Außendurchmesser von höchstens 0,5 mm, insbesondere höchstens 0,48 mm, insbesondere höchstens 0,47 mm aufweist und der Katheter (10) einen Außendurchmesser von 0,6 mm bis 0,8 mm, insbesondere von 2,5 F aufweist.

5. Temperaturmessvorrichtung nach einem der vorhergehenden Ansprüche,
**dadurch gekennzeichnet, dass**
der Temperaturmesssensor (11), insbesondere der Messfühler (12) wenigstens eine Kontaktfläche (14) für den Kontakt mit Blut aufweist, wobei die Kontaktfläche (14) einen abgegrenzten Außenflächenabschnitt des Katheters (10) bildet.

6. Temperaturmessvorrichtung nach Anspruch 4,
**dadurch gekennzeichnet, dass**
dass der Katheter (10) aus einem ersten Material und der Außenflächenabschnitt aus einem zweiten Material gebildet sind, das sich vom ersten Material unterscheidet.

7. Temperaturmessvorrichtung nach Anspruch 4 oder 5,
**dadurch gekennzeichnet, dass**
die Kontaktfläche (14) seitlich in oder an einer Außenwand (15) des Katheters (10) ausgebildet ist und/oder die Kontaktfläche (14) eine distale Spitze (21) des Katheters (10) bildet.

8. Temperaturmessvorrichtung nach einem der vorhergehenden Ansprüche,
**dadurch gekennzeichnet, dass**
ein Führungsdraht (19) in Längsrichtung durch den Katheter (10) bewegbar, insbesondere im Katheter (10) angeordnet ist und/oder der Führungsdraht (19) in einem dritten Lumen (20) oder im gemeinsamen Lumen (18) angeordnet ist.

9. Temperaturmessvorrichtung nach Anspruch 8,
**dadurch gekennzeichnet, dass**
der im gemeinsamen Lumen (18) angeordnete Führungsdraht (19) fixiert ist, insbesondere durch den als Schrumpfschlauch ausgebildeten Katheter (10) fixiert ist..

10. Temperaturmessvorrichtung nach einem der vorhergehenden Ansprüche,
**dadurch gekennzeichnet, dass**
der Temperaturmesssensor (11), insbesondere der Messfühler (12) wenigstens einen Messdraht (24) oder ein PTC-Sensorelement oder ein NTC-Sensorelement oder ein Dünnschicht-Sensorelement aufweist.

11. Temperaturmessvorrichtung nach einem der vorhergehenden Ansprüche,
**dadurch gekennzeichnet, dass**
der Katheter (10) wenigstens zwei Temperaturmesssensoren (11), insbesondere an unterschiedlichen Positionen, aufweist.

12. Temperaturmessvorrichtung nach einem der vorhergehenden Ansprüche,
**dadurch gekennzeichnet, dass**
der Katheter (10) eine Länge von wenigstens 0,6 m, insbesondere wenigstens 0,7 m, insbesondere wenigstens 0,8 m, insbesondere wenigstens 0,9 m, insbesondere wenigstens 1,0 m, insbesondere wenigstens 1,1 m, insbesondere wenigstens 1,2 m aufweist.

13. Temperaturmessvorrichtung nach einem der vorhergehenden Ansprüche,
**dadurch gekennzeichnet, dass**
der Katheter (10) für intracerebrale Anwendungen eine Länge von wenigstens 1,3m, insbesondere wenigstens 1,4m, insbesondere wenigstens 1,5m aufweist und/oder der Katheter (10) für intracerebrale Anwendungen einen Außendurchmesser von höchstens 0,8mm, insbesondere höchstens 0,7mm, insbesondere höchstens 0,6mm, insbesondere höchstens 0,5mm, insbesondere höchstens 0,4mm, insbesondere höchstens 0,3mm aufweist.

14. Kühlkatheter mit einer Temperaturmessvorrichtung nach einem der vorhergehenden Ansprüche und einem Kühlelement (25), wobei die Temperaturmessvorrichtung in einem Lumen des Kühlkatheters derart längsbeweglich angeordnet ist, dass der Temperaturmesssensor (11) in distaler Richtung vom Kühlelement (25) beabstandet angeordnet werden kann.

15. Kühlkatheter nach Anspruch 15,
**dadurch gekennzeichnet, dass**
ein expandierbares Rekanalisationselement (26) im Katheter (10) derart längsbeweglich angeordnet ist, dass das Rekanalisationselement (26) in distaler Richtung vom Kühlelement (25) beabstandet angeordnet werden kann, wobei insbesondere das Rekanalisationselement (26) und die Temperaturmessvorrichtung relativ beweglich zueinander sind.

16. Verfahren zur intravaskulären Temperaturmessung von Blut, bei dem eine Temperaturmessvorrichtung nach einem der vorhergehenden Ansprüche durch einen Kühlkatheter soweit vorgeschoben wird, dass der Temperaturmesssensor (11) distal von einem Kühlelement (25) des Kühlkatheters positioniert wird.
